# EUROPEAN PATENT APPLICATION

(11) **EP 3 662 843 A1**
(43) Date of publication of application: **10.06.2020**
(21) Application number: 18210315.0
(22) Date of filing: 05.12.2018
(51) Int. Cl.: A61B 10/00

(54) **KIT FOR THE SAMPLING OF BIOLOGICAL LIQUID SUCH AS URINE AND METHOD UTILIZING SUCH KIT**

(71) Applicant: PALMA GROUP SA, 1700 Fribourg (CH)
(72) Inventor: PORVAN, Pavlo, 1700 Fribourg (CH)
(74) Representative: Bugnion Genève

(57) **Abstract**

The invention relates to a kit (1) for the sampling of biological liquid in particular urine comprising: a flexible accumulator (2) of biological liquid adapted to collect the liquid comprising at least one liquid absorbing layer (10) and at least one support layer (11) attached thereto; at least one closed recipient (3) comprising a tubular container (5) with a tubular sidewall portion (6) and a bottom portion (7) forming an elongated tubular cavity (9) and a closing lid (5) sealingly connected to the sidewall portion (6); wherein the accumulator (2) is configured for being inserted in the tubular cavity (9) of the container, wherein the recipient (3) comprises holding means (12) configured for maintaining the accumulator in position in the recipient and distant from the bottom portion (7) of the container.

## Description

### FIELD OF THE INVENTION

The invention relates to the field of the sampling of biological liquid in particular urine for applications such as the medical research on human health or the diagnostic of human diseases or nutritional deficiencies such as for infant or elderly people.

### BACKGROUND OF THE INVENTION

Despite the wide range of diagnostic methods and equipment, there is still an unmet need for convenient and hygienically safe tools enabling to collect biological liquids in particular urine from human body in a manner that enables to reliably perform analysis and/or diagnostics based on samples of the liquid.

A problem of sampling urine, in amount sufficient for biological analysis, lies in that it is not easy to collect such liquid on demand especially from infants or young children or functionally limited people such as disabled or elderly persons.

A solution described in WO2017168388 consists in proposing a solution of diaper for the selection of biological samples comprising an accumulator formed as an absorbing band removably and externally connected to the diaper, in particular positioned about a through-hole collecting area of the diaper.

In co-pending international patent application PCT/IB2017/056789, a method for the identification of compounds of human urine and/or determination of analytical parameters such as pH is described. The method utilizes a kit comprising the accumulator of WO2017168388 and urinary indicating bands comprising multiple indicators, including colour reagents, which are wetted by contact with the urine accumulator. The urinary indicating bands can be held on an analysis device which bears colorimetric scales to compare the change of colour of the indicators of the band with the colorimetric scale to analyse the compounds and analytical parameters.

However, other more sophisticated analysis such as microscopy, chromatography, or others, require the collection of samples of a minimal volume of liquid. Urine is composed of more than 15 analytes (glucose, ketones, albumin, leucocyte, erythrocytes, etc.) which are interesting for fine analysis. Todays' analysis provide a loss of information due to the techniques of sampling which are not reliable and safe enough.

Therefore, there is a need for a solution to collect enough biological liquid in particular urine with the convenience of the accumulator of WO2017168388 and also in a manner that enables the subsequent in-depth analysis of the liquid such as microscopy and the like to be successfully carried out.

In particular, there is a need for collecting and sampling biological liquid in sufficient amount, rapidly, safely and without risk of cross-contamination.

### BRIEF SUMMARY OF THE INVENTION

The invention aims at providing solutions to the above-identified problems.

In particular, the invention relates to a kit for the sampling of biological liquid in particular urine comprising:
a flexible accumulator of biological liquid adapted to collect the liquid from a human body formed as an elongated band comprising at least one liquid absorbing layer and at least one support layer attached to the liquid absorbing layer;
at least one closed recipient comprising a tubular container with a tubular sidewall portion and a bottom portion forming an elongated tubular cavity and a closing lid sealingly connected to the open end of the sidewall portion opposite the bottom portion;
wherein the accumulator is configured for being inserted in the tubular cavity of the container through the open end when the closing lid is removed;
wherein the recipient further comprises holding means configured for maintaining the accumulator in position in the recipient and distant from the bottom portion of the container upon re-closing of the container to enable liquid previously absorbed by the accumulator to be collected in the recipient in a reserved volume part of the cavity not occupied by the accumulator, between the bottom portion of the recipient and the accumulator, when the recipient is centrifuged in a centrifugal liquid collecting machine.

Preferably, at least the length of the absorbing layer of the accumulator is shorter than the internal depth of the tubular cavity. More preferably, the length of the accumulator is shorter than the internal depth of the tubular cavity. Such configuration ensures that at least the part of the accumulator which is charged with biological liquid can be entirely inserted in the recipient in non-folded manner in the axial direction of the recipient.

Therefore, the invention provides an easy and reliable solution to sample liquid such as urine while making sure a sufficient amount of liquid is separated from the accumulating element well reserved in a safe and uncontaminated volume before it can be analysed by microscopy or other tools. Generally, the recipient is capable to hold the accumulator while avoiding it to collapse in the reserved part even at elevated centrifugal speeds, e.g. in a range of 500-800 rpm.

The "length" of the accumulator or of the absorbing layer represents the maximal dimension in the longitudinal direction of the considered element that corresponds to the direction of insertion of the accumulator in the recipient.

In particular, the internal surfaces and internal volume of the closed recipient of the kit is preferably sterile before the first opening to receive the accumulator inside. The recipient can be made sterile by any suitable non-intrusive sterilisation means such as by heat and/or irradiation, e.g. gamma rays.

The closing lid may be any kind of seal member such as a multiple-use plastic lid and/or a single-use sealing membrane. Preferably, the lid is formed as a plastic cap and preferably has reversible connection means such as a thread and the open end of the container has a complementary connection means such as a thread.

Preferably, the holding means are configured to define a reserved part of the volume of the cavity not occupied by the accumulator when held in the recipient of between 1 and 10 ml and/or of between 0.5 and 2.5 cm long. Most preferably, the reserved part of the volume is between 1 and 10 ml more preferably between 1 and 3 ml and/or between 0.8 and 2.5 cm more preferably between 1 and 1.5 cm. The kit is thus well adapted to very small quantities of liquid to be collected and reserved.

Preferably, the length of the absorbing layer is shorter than the length of the volume part of the cavity situated above the holding means. As a result, the absorbing layer can be entirely contained in the recipient once closed thereby ensuring no spillage of liquid outside the recipient during centrifugation.

In a preferred mode the holding means comprises at one abutment member located in the reserved volume part of the cavity and sized to hold the accumulator above the bottom portion of the container. Preferably, the accumulator is held at a distance between 1 and 3 cm above the bottom portion of the container.

More specifically, the holding means comprises a series of ribs provided at the internal surface of the tubular sidewall portion. Most preferably, the ribs protrudes inwardly from the tubular internal surface and are distributed over the circumference of the tubular internal surface. Such ribs configuration provides the advantage to offer an efficient support for the accumulator by well distributing reaction force during the centrifugation operation and so prevents the accumulator from collapsing in the recipient.

Preferably, the ribs are oriented along the axial direction of the elongated tubular cavity and end by free edges in direction of the open end of the container. The neighboured ribs may be distanced one another from a linear distance of less than 2 cm, preferably less than 1.5 cm, most preferably between 1.2 and 0.5 cm, to hold the accumulator. In particular, the support layer at least when the accumulator is partially rolled in the recipient about the longitudinal axis.

In particular, the tubular portion of the container has a diameter comprised between 1 and 5 cm, more preferably between 1 and 3.5 cm, most preferably between 1.5 and 2.5 cm.

The accumulator is relatively flexible although resistant to traction forces and to tearing. The resistance to tearing of the accumulator is preferably obtained by the mechanical properties of the support layer. In particular, the support layer is made of a material having higher tear strength than the material of the absorbing layer.

The configuration of the ribs and/or diameter of the recipient enables to insert the accumulator in an at least partially rolled fashion about its longitudinal axis so that its rigidity is enhanced to prevent it from collapsing during centrifugation. Furthermore, this arrangement of properly distanced ribs reduces the risk of the flexible accumulator to collapse while providing enough reserved volume for liquid.

In another mode, the abutment member is a removable elongated insert of smaller length than the internal depth of the cavity, e.g. with a transversal cross-section having the general form or configuration of a cross, a full or partial tube or a transversal grid.

The insert is preferably in place in the closed recipient and is also sterile. The insert may be made of a rigid polymer material such as polypropylene or polyethylene. For example, the insert can be a small grid arranged for separating the volume of the cavity in two parts; an upper part of the cavity for receiving the accumulator and a lower part of the cavity for forming the reserve for the centrifuged liquid.

In another mode, the holding means comprise an elongated rigid support platform comprising a holding surface configured for receiving the accumulator and comprising attachment means configured for being attached to the internal side of the lid, the support platform having a length shorter than the internal depth of the tubular cavity to be fixed to the lid at distance from the bottom portion of the recipient when closed.

In particular, the support platform is a rectangular plate-like member larger than the width of the accumulator but smaller than the width or diameter of the tubular sidewall portion.

This platform configuration provides the advantage to avoid rolling the accumulator before inserting it inside the recipient. The support platform can be introduced in the container without being deformed while holding the accumulator, preferably flat. As a result, the potential loss of liquid is reduced, as the absorbing layer is kept flat and uncompressed. The risk of contamination is also limited.

Preferably, the support platform comprises at least one retaining member configured for holding the accumulator at least in the axial direction of the container, in particular with the support layer being retained in contact on the support platform.

Preferably, the retaining member may comprise any one or more of: a slider on the platform, an attachment pin, a mechanical adhesive or a chemical adhesive and combinations thereof. The slider(s) may advantageously be formed integrally with the platform in a single piece, such as made out of rigid plastic.

With such arrangement of the retaining means, the accumulator is fixed on the platform and is prevented from moving because of the centrifugation forces.

Preferably, the liquid absorbing layer can be a relatively thick compressible layer of fibres, e.g. 0.5 to 2 cm, preferably synthetic fibres such as made of viscose and polystyrol. The support layer or layers may be a traction resistant layer of medical paper or woven layer. The layer can be made of polymer and/or paper. In particular, the support layer can comprise a polymer-coated film of cellulose or paper. The support layer may have a thickness between 0.2 and 1 mm.

The accumulator may have a length between 5 and 10 cm and a width between 2 and 4 cm.

The accumulator may optionally comprise additional layers such as an attachment layer on top of the support layer and opposite to the liquid absorbing layer. The attachment layer may have a non-permanent adhesive inner surface configured for connecting the support and absorbing layers in a removable manner to the diaper.

The support layer and/or attachment layer may define the greatest length and width of the accumulator. The liquid absorbing layer may have a length and width, e.g. 10 to 50 % shorter, than the length and width of the support layer. The support layer may have a length and width shorter, e.g. 10 to 70% shorter than the length and width of the attachment layer.

It should be noted that the attachment layer may also be part of the diaper. In such case, the accumulator only comprises the liquid absorbing layer and the support layer attached one another.

Furthermore, the kit may comprises a diaper comprising at its external side a collecting area through which the accumulator is removably fixed by means of an attachment layer of dimensions larger in all directions than the surface of the support layer of the accumulator to protect the accumulator in the collecting area during collection of the biological liquid, the attachment layer comprising a reversible, pressure sensitive adhesive inner surface arranged for holding in a removable manner the accumulator in the collecting area.

In particular, the collecting area may be .g. a hole through the diaper or a liquid permeable wall area in the thickness of the diaper.

The invention further relates to a method for collecting biological liquid in particular urine from the kit as aforementioned, wherein after the absorbing layer of the accumulator is charged with liquid, the accumulator is inserted in the container while being held by the holding means and at distance from the bottom portion of the container, the recipient is closed by the closing lid and the recipient is centrifuged in a liquid centrifugal machine to collect liquid in the reserved volume part of the elongated cavity of the container, while the holding means hold the accumulator in place thereby keeping the reserved part of the cavity unoccupied by the accumulator when collecting liquid therein.

Preferably, the reserved part of the cavity is of about 1 to 5 ml.

Preferably, the recipient is closed by the closing lid, with the interior cavity being sterile before opening, preferably by removal of the closing lid and insertion of the accumulator in the container and subsequent closing, preferably by the closing lid, for sampling of the liquid by centrifugation. Preferably, the closing lid is threaded in a removable fashion to a threaded portion of the container to close its open end.

### BRIEF DESCRIPTION OF THE FIGURES BREVE DESCRIPTION DES FIGURES

Features described in relation to one aspect may equally be applied to other aspects of the invention.

Embodiments of the invention will now be described, by way of preferred examples only, with reference to the accompanying drawings, in which:
Fig. 1 shows a first embodiment of the kit of the invention;
Fig. 2 shows the recipient of the kit of Fig. 1;
Fig. 3 is a longitudinal cross-section view of the accumulator of the kit of the invention;
Fig. 4 is a transversal cross-sectional view of the container of the recipient of the kit according to the embodiment of Fig. 1;
Fig. 5 illustrates the assembling of the kit of Fig. 1 for liquid sampling purpose;
Fig. 6 is a schematic representation of the centrifugation of the kit for collecting biological liquid in the container of the kit;
Fig. 7 shows a second embodiment of the kit of the invention;
Fig. 8 shows the accumulator retained on the support platform of the kit of Fig. 7;
Fig. 9 is a cross-section view of the container of the kit according to a first variant of the mode of Fig. 1;
Fig. 10 is a cross-section view of the container of the kit according to a second variant of the mode of Fig. 1;
Fig. 11 is a cross-section view of the container of the kit according to a third variant of the mode of Fig. 1;
Fig. 12 is a schematic representation of a diaper and accumulator of the kit of the invention for collecting the urine from a human body;
Fig. 13 is a plane view of the diaper and accumulator of Fig. 12.

### DETAILED DESCRIPTION OF THE DRAWINGS

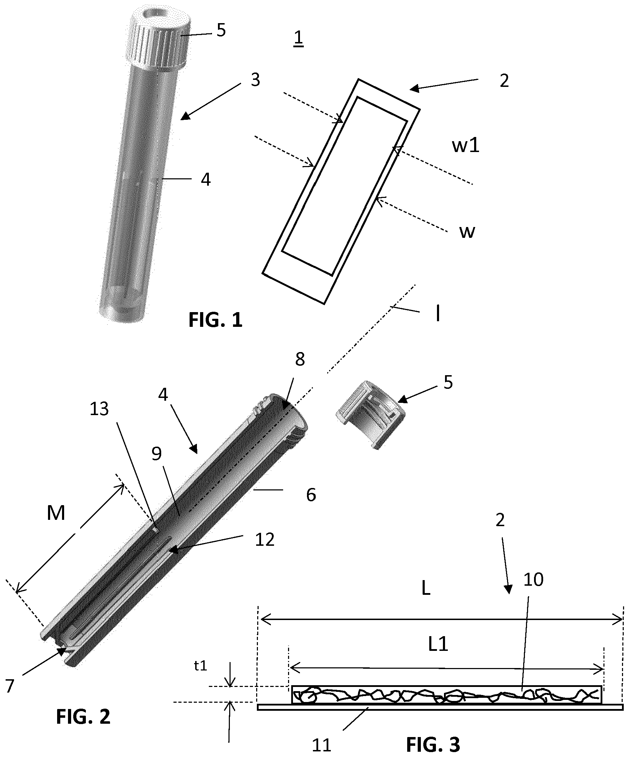

A first mode of the sampling kit 1 of the invention is illustrated in Fig. 1 to 6. The kit comprises a flexible accumulator 2 for collecting biological liquid, in particular urine, on a human body and a closed recipient 3 adapted for receiving the flexible accumulator and for sampling liquid collected by the accumulator in a centrifugation medical device.

The recipient 3 comprises an elongated tubular container 4 and a closing lid 5. In the closed configuration before first use, the interior surfaces of the recipient are preferably sterile. The process of sterilization can occur during the manufacturing of the container such as by gamma irradiation or other suitable techniques. The closing lid 5 is connected to the container in removable and repeatable manner such as by a threading assembly. The connection is preferably a sealing connection in the sense that the interior environment can be maintained sterile during an extensive period of storage.

In a possible mode, the container can be closed in sterile fashion before its first opening by means of a closing lid formed by at least one peelable sealing membrane. A combination of a threaded lid associated to or on top of a peelable sealing membrane is also a possible closing lid.

As shown in Fig. 2, the tubular container 4 comprises an elongated tubular sidewall portion 6, a bottom portion 7 at a first end of the tubular portion and an open end 8. The tubular container is preferably cylindrical. However, other forms are possible. The elongated tubular sidewall portion and bottom portion define an elongated internal cavity 9 extending along a longitudinal axis I.

An example of liquid accumulator 2 is represented on Fig. 3. It comprises at least one absorbing layer 10 and a support layer 11 ; both are made of flexible materials so that the accumulator can be conformed to fit to a diaper for collecting urine. In particular, the liquid absorbing layer is designed to absorb a sufficient quantity of liquid, more preferably between 1 and 5 ml. The liquid absorbing layer is preferably made of hydrophilic material an/or that can store liquid. The layer can be formed of fibres such as a nonwoven or a fabric. A suitable material can be a combination of viscose and polystyrol. The thickness t1 can be of 1 to 3 mm, its length L1 may be between 3 and 8 cm and its width w1 can be of 1 to 3 cm.

The support layer 11 is designed to avoid the absorbing layer to tear apart. It can be a tear resistant film made of polymer and/or cellulose or paper. Its dimensions (L, w) are preferably higher than the dimensions (L1, w1) of the absorbing layer 10. In particular, its length L can be of 5 to 12 cm and its width w can be of 1.5 to 4 cm.

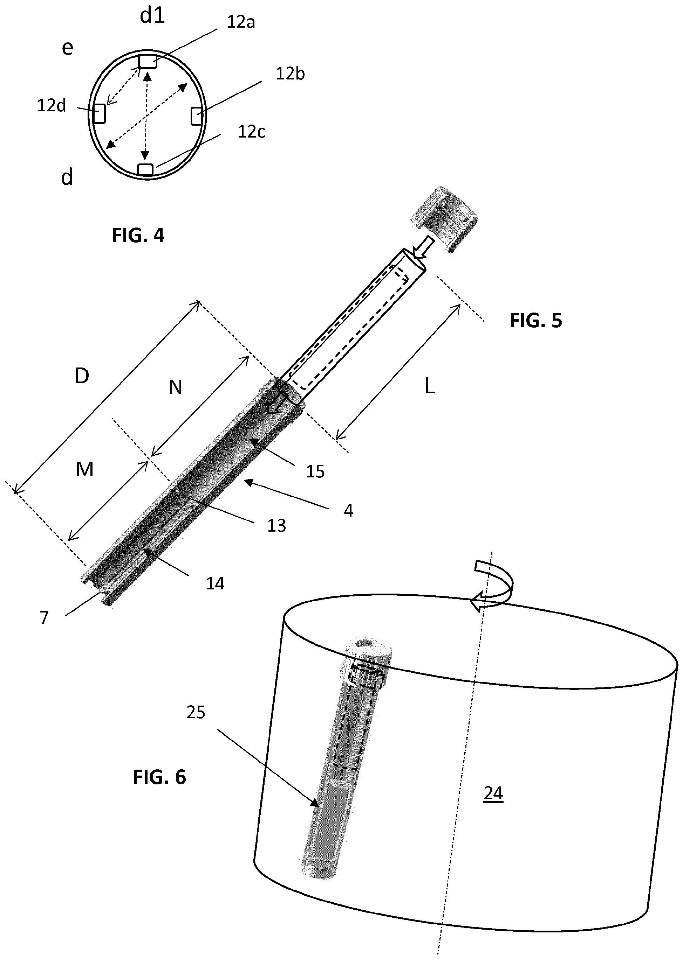

According to an aspect of the invention, the tubular recipient 3 comprises holding means designed for holding the accumulator 2 when it is inserted in the container, such holding means being such that it avoids the accumulator from collapsing during centrifugation. In this mode, the holding means comprises a series of holding ribs 12, 12a, 12b, 12c, 12d oriented in the longitudinal direction I and protruding on the internal surface of the sidewall portion 6. The ribs comprise free edges 13; at least some of them being located at distance M from the bottom portion 7 such that a reserved volume part 14 of the cavity is provided between the bottom portion and an upper cavity portion 15 without ribs. The diameter d of the sidewall portion of the container and the distance M are thus defined to create a volume, which is commensurate with the amount of liquid contained in the accumulator and/or the amount of liquid required for subsequent analysis. The diameter d of the container is preferably smaller than the width w of the accumulator, preferably 1.5 to 3 times smaller, such that the accumulator needs to be rolled about its longitudinal axis to be properly inserted in the container open as illustrated in Fig. 5. As a result, by virtue of its axially rolled configuration, the accumulator becomes more rigid than if inserted flat or unrolled. Moreover, due to the natural tendency of the accumulator to return to its unrolled position, the accumulator applies to the periphery of the tubular portion and so abuts on the series of holding ribs 12 in such rolled configuration. It is also to be noted that the length N of the upper volume of the cavity of the container which is not occupied by the holding means is preferably dimensioned to be shorter than the length L1 of the absorbing layer 10 so that the absorbing layer can be inserted in the container fully and without being folded in axial direction of the recipient. The length L of the accumulator is also preferably smaller than the length N of the upper volume but it can also be slightly longer and e.g. can be folded at the open end 8 and e.g. can be pinched between the container and the closing lid (as schematically illustrated in Fig. 6).

The number of ribs can be variable, preferably between 2 and 10. They are preferably distributed over the circumference of the internal surface of the sidewall portion. They may end before the bottom portion or may extend down to the bottom portion. The neighbouring ribs may be distanced from a small distance such as 3 to 5 mm; whereas the difference between the internal diameter of the container d and the longest distance between ribs d1 (i.e. "d-d1") is designed to provide sufficient support to the accumulator. For example, the radial thickness of each rib is between 0.8 and 2 mm. Preferably, the container and ribs are formed as a single integral piece of polymer such as by blow moulding, extrusion or injection.

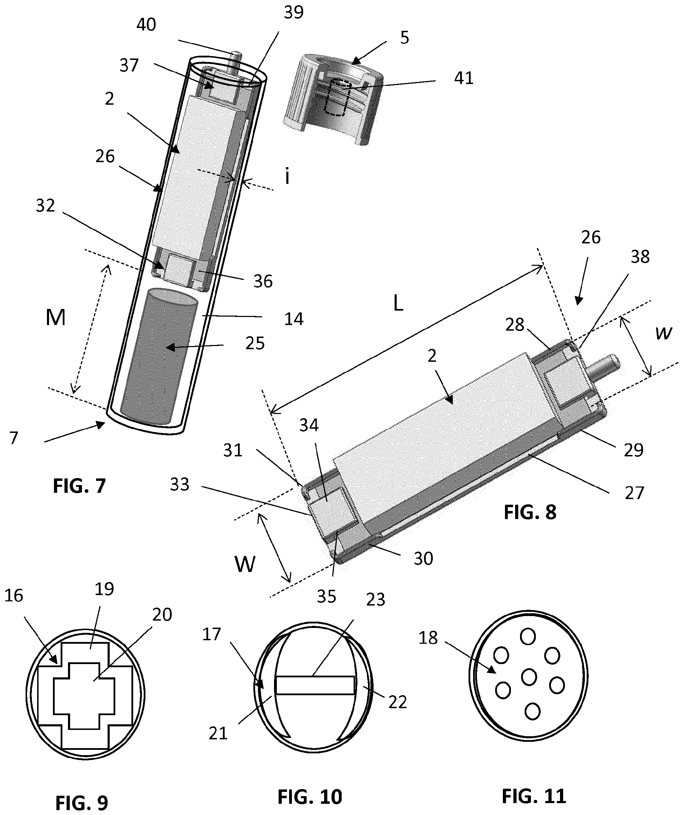

Other shapes of the holding means are represented as possible examples in Figs. 9, 10 and 11. The holding means may be at least one insert 16, 17, 18 which is separable from the container and dimensioned to fit in the cavity while leaving a reserved part 14 for liquid to settle.

For example, in Fig. 9, the insert 16 forms a hollow profile with a loop-shaped holding wall 19 and a central aperture 20. The hollow profile may have many various cross-section shapes such as cross-like shape (as represented) or cylindrical or polygonal shapes.

In Fig. 10, the holding means comprises an insert 17 formed of two moon-like shaped parts 21, 22 connected by a central link 23. The parts can form together a single integral piece or alternatively separate pieces.

In Fig. 11, the holding means comprises an aperture grid. The grid can separate the reserved part of cavity for the liquid from the accumulator placed above in the rest of the cavity of the container.

Once closed or re-closed by the threaded lid with the accumulator fitted in, the recipient can be centrifuged in a conventional medical centrifugal machine for separating the liquid from the accumulator. The centrifugal speed can be controlled between 200 and 800 rpm between e.g. 20 seconds and 5 minutes, to force liquid 25 to settle in the reserved part of the volume beside the bottom portion. The volume of sampled liquid may vary from 1 to 5 ml depending on the capacity of the accumulator and the capacity of the reserved part 14.

A second embodiment is illustrated in relation to Figs. 7 and 8. In this case, the accumulator 2 is held by a support platform 26 allowing the accumulator to be inserted flat inside the recipient. Thus, the support platform 26 comprises a support surface 27 for receiving the support layer 11 of the accumulator. The width W of the platform is chosen slightly wider than the width w of the accumulator and lateral guiding ridges 28, 29, 30, 31 may be provided at the side and/or corners of the platform to facilitate positioning and contact of the accumulator on the support surface 27. Preferably, a retaining member 32 such as a slider may be provided at the distal axial end of the support platform to prevent the accumulator to move axially towards the bottom portion of the container during centrifugation. The slider may be formed as a L-shaped element with a transversal stopping wall 33, a top stopping wall 34 and with one or two opposite transversal openings 35 for enabling a first end 36 of the support layer of the accumulator to be inserted in. Preferably, a second slider 37 is provided at the proximal end 38 of the support platform for grabbing the second end 39 of the support layer. As a result, the accumulator is axially stopped and retained on the platform. Moreover, due to the small differential transversal dimensions, [i= d (minus) W] between the internal diameter of the container and the width of the platform, preferably smaller than 0.5 cm, the accumulator is not allowed to move transversally to such an extent that it can disengage from the retaining members 32, 37.

The support platform can be attached to the recipient, either to the container or lid, by any suitable means, provided the support platform is maintained at distance from the bottom portion 7 of the tubular container such as to provide the reserve part 14 of volume for liquid. For example, the support platform comprises a connection member 40, e.g. a pin, at its proximal end which engages a complementary connection member 41, e.g. a hose provided at the interior side of the lid 5. The pin and hose may be assembled e.g. by force fitting or screwing or any other suitable assembly means resisting centrifugation.

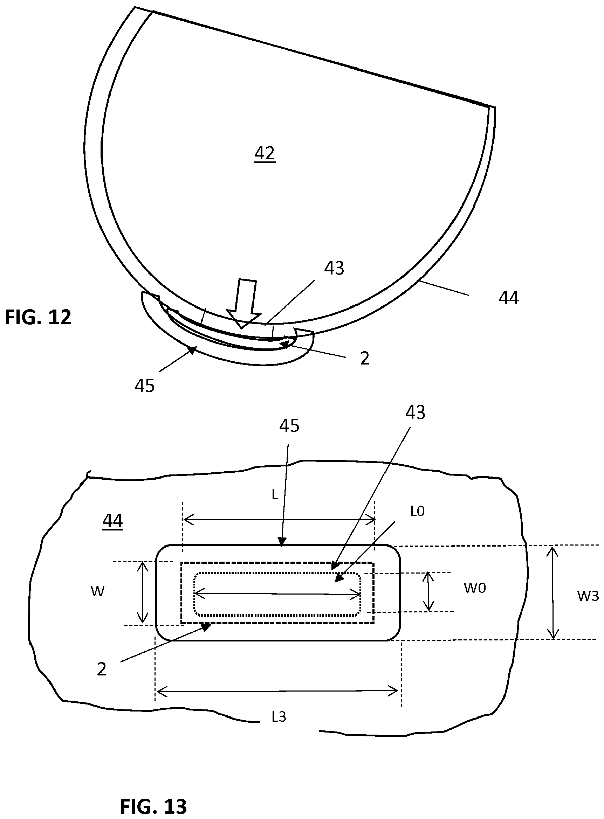

The kit 1 of the invention may encompass a collecting diaper 42 as illustrated in Fig. 12; the diaper comprising a collecting area 43 opens towards the external side 44 of the diaper. The collecting area 43 may be a e.g. rectangular, hole through the thickness of the diaper or a locally liquid permeable wall of the diaper enabling liquid, in particular urine to pass from the inside of the diaper through the collecting area and to the absorbing layer of the accumulator as shown by arrow in Fig. 12. For example, a permeable wall can be formed of a thin tissue and/or porous fibres. The accumulator 2 is designed and dimensioned (L, w) to externally cover the collecting area (of dimensions L0, w0) of the diaper, as illustrated in Fig. 13. The accumulator can be maintained in place on the diaper during the collection time by an attachment layer 45 of even larger dimensions, (L3, w3) than the dimensions of the accumulator (L, w). The dimension of the absorbing layer (L1, w1) (not represented) is preferably slightly larger than the dimensions (L0, w0) of the collecting area to prevent leakage of liquid out between the accumulator and the diaper. The attachment layer 45 preferably comprise a reversible pressure sensitive mechanical and/or chemical adhesive which at least makes the attachment layer adhere to the external surface of the diaper, and preferably also to the support layer 11 of the accumulator. A mechanical adhesive may be e.g. a respectively buckle surface or anchoring surface connecting to a respectively anchoring surface or buckle surface of the diaper and/or accumulator, known as for instance, a Velcrotype connection means. A reversible chemical adhesive may be any adhesive such as methylcellulose and the like.

The invention further relates to a method for sampling a biological liquid, in particular urine, by means of the kit as described generally or in any of the preceding preferred modes.

The method comprises the following steps:
- collecting the biological liquid in the accumulator 2 by associating the accumulator with a collecting diaper 42,
- providing the closed recipient 3 preferably internally sterile, opening it and inserting the accumulator 2 charged with biological liquid in the recipient and closing it; while the accumulator being held by the holding means 12, 16, 17, 18, 26 in the container to define a reserved volume part 14 of the recipient between the bottom portion 7 of the container and the accumulator 2 fitted in the container;
- transferring liquid from the accumulator 2 to the reserved volume part 14 of the recipient, preferably by centrifugation of the recipient,
- optionally, storing the recipient 3 closed for subsequent analysis of the reserved liquid 25.

## Claims

1. Kit (1) for the sampling of biological liquid in particular urine comprising:
a flexible accumulator (2) of biological liquid adapted to collect the liquid from a human body formed as an elongated band comprising at least one liquid absorbing layer (10) and at least one support layer (11) attached thereto;
at least one closed recipient (3) comprising a tubular container (5) with a tubular sidewall portion (6) and a bottom portion (7) forming an elongated tubular cavity (9) and a closing lid (5) sealingly connected to the open end (8) of the sidewall portion (6) opposite the bottom portion (7);
**characterized in that** the accumulator (2) is configured for being inserted in the tubular cavity (9) of the container through the open end (8) when the closing lid is removed;
and **in that** the recipient (3) further comprises holding means (12, 12a-12d, 16, 17, 18, 26) configured for maintaining the accumulator in position in the recipient and distant from the bottom portion (7) of the container upon re-closing of the container to enable liquid previously absorbed by the accumulator to be collected in the recipient in a reserved part of the volume not occupied by the accumulator, between the bottom portion of the recipient and the accumulator, when the recipient (3) is centrifuged in a centrifugal liquid collecting machine (24).

2. Kit according to claim 1, wherein the length (L) the length (L1) of the absorbing layer of the accumulator is shorter than the internal depth (D) of the tubular cavity (9).

3. Kit according to claim 1 or 2, wherein the holding means (12, 12a-12d, 16, 17, 18, 26) are configured to define a reserved volume part (14) not occupied by the accumulator (2) when held in the recipient; said part (14) being sized between 1 and 10 ml and/or of between 0.5 and 2.5 cm long.

4. Kit according to any one of claims 1 to 3, wherein the holding means comprises at one abutment member (12, 12a-12d, 16, 17, 18) located in the reserved volume part (14) of the cavity and sized to hold the accumulator (2) above the bottom portion (7) of the container.

5. Kit according to claim 4, wherein the holding means comprises a series of ribs (12, 12a-12d) provided at the internal surface of the tubular sidewall portion (6).

6. Kit according to claim 5, wherein the ribs (12, 12a-12d) are oriented along the axial direction (I) of the elongated tubular cavity (9) and end by free edges (13) in direction of the open end (8) of the container and the neighboured ribs (12a, 12b, 12c, 12d) are distanced one another from a linear distance (e) of less than 2 cm, to hold the accumulator, in particular, the support layer (11) at least when the accumulator is partially rolled in the recipient about the longitudinal axis (I).

7. Kit according to claim 4, wherein the abutment member is a removable elongated insert (16, 17, 18) of smaller length than the internal depth (D) of the cavity (9), e.g. with a transversal cross-section having the general form or configuration of a cross, a full or partial tube or a transversal grid.

8. Kit according to any one of claims 1 to 3, wherein the holding means comprises an elongated rigid support platform (26) comprising a holding surface (27) configured for receiving the accumulator (2) and comprising attachment means (40) configured for being attached to the internal side of the lid (5), the support platform having a length (L) shorter than the internal depth (D) of the tubular cavity to be fixed to the lid (5) at distance from the bottom portion of the recipient (2) when closed.

9. Kit according to claim 8, wherein the support platform (26) is a rectangular plate-like member larger than the width (w) of the accumulator but smaller than the width or diameter (d) of the tubular sidewall portion (6).

10. Kit according to claims 8 or 9, wherein the support platform (26) comprises at least one retaining member (32, 37) configured for holding the accumulator (2) at least in the axial direction (I) of the container, in particular with the support layer (11) being retained in contact on the support platform.

11. Kit according to claim 10, wherein the retaining member (32) comprises any one or more of: a slider on the platform, an attachment pin, a mechanical adhesive or a chemical adhesive and combinations thereof.

12. Kit according to any one of the preceding claims, wherein it further comprises a diaper (42) comprising at its external side (44) a collecting area (43), e.g. a hole or liquid permeable wall area, through which the accumulator (2) is removably fixed by means of an attachment layer (45) of dimension (L3, W3) larger in all directions than the surface of the support layer (11) of the accumulator to protect the accumulator in the collecting area (43) during collection of the biological liquid, the attachment layer (45) comprising a reversible, pressure sensitive adhesive inner surface arranged for holding in a removable manner the accumulator(2) in the collecting area (43).

13. Method for collecting biological liquid in particular urine from the kit (1) according to any of the preceding claims, wherein after the absorbing layer (10) of the accumulator (2) is charged with liquid, the accumulator is inserted in the container (4) while being held by the holding means (12, 12a-12d, 16, 17, 18, 26) and at distance (M) from the bottom portion (7) of the container, the recipient (2) is closed by the lid (5) and the recipient is centrifuged in a liquid centrifugal machine (24) to collect liquid in the reserved part (14) of the elongated cavity (9) of the container, while the holding means hold the accumulator in place thereby keeping the reserved part of the cavity (9) unoccupied by the accumulator (2) when collecting liquid therein.

14. Method according to claim 13, wherein the reserved part (14) of the cavity (9) is of about 1 to 5 ml.

15. Method according to claim 13 or 14, wherein the recipient (3) is closed by the closing lid (5) with the interior cavity (9) being sterile before opening, preferably by removal of the closing lid (9) and insertion of the accumulator (2) in the container (4) and subsequent closing, preferably by the closing lid (9), for sampling of the liquid by centrifugation.
